# EUROPEAN PATENT APPLICATION

(11) **EP 4 292 586 A1**
(43) Date of publication of application: **20.12.2023**
(21) Application number: 23179189.8
(22) Date of filing: 14.06.2023
(51) Int. Cl.: A61K 9/20, A61K 31/165

(54) **GUANFACINE HYDROCHLORIDE CONTAINING PHARMACEUTICAL PREPARATION**

(30) Priority: 15.06.2022 US 202263352282 P; 08.06.2023 JP 2023094807
(71) Applicant: Sawai Pharmaceutical Co., Ltd., Yodogawa-ku Osaka-shi Osaka 532-0003 (JP)
(72) Inventor: OKADA, Taro, Osaka, 5320003 (JP); KIMATA, Ryota, Osaka, 5320003 (JP); YOSHIHARA, Naoki, Osaka, 5320003 (JP)
(74) Representative: dompatent von Kreisler Selting Werner - Partnerschaft von Patent- und Rechtsanwälten mbB

(57) **Abstract**

An object of the present invention is to provide a guanfacine hydrochloride preparation in which the increase of related substances with time due to hydrolysis is suppressed. According to an embodiment of the present invention, there is provided a guanfacine hydrochloride preparation containing guanfacine hydrochloride, one or more stability-improving additives selected from a group consisting of potassium dihydrogen phosphate, phosphoric acid, sodium dihydrogen phosphate anhydrous, carmellose, microcrystalline cellulose, carmellose calcium, low-substituted hydroxypropylcellulose, hypromellose phthalate, and glacial acetic acid; and hypromellose acetate succinate; and one or more additives selected from a group consisting of aspartic acid, succinic acid, alginic acid, and glutamic acid.

## Description

### TECHNICAL FIELD

An embodiment of the present invention relates to a guanfacine hydrochloride containing pharmaceutical preparation.

### BACKGROUND ART

A guanfacine hydrochloride containing pharmaceutical preparation (N-Amidino-2-(2,6-dichlorophenyl)acetamide monohydrochloride) is a selective α_{2A} adrenergic receptor agonist and has been used as a therapeutic for attention deficit hyperactivity disorder (AD/HD).

In a guanfacine hydrochloride preparation, an increase in the amount of related substances with time due to hydrolysis is observed, and in Patent Literature 1, the production of hydrolysate of guanfacine hydrochloride is suppressed by blending a specific organic acid and/or an enteric base.

### CITATION LIST

### PATENT LITERATURE

Patent Literature 1: Japanese laid-open patent publication No. 2023-035359

### SUMMARY OF INVENTION

### TECHNICAL PROBLEM

An object of an embodiment of the present invention is to provide a guanfacine hydrochloride preparation in which the increase of related substances with time due to hydrolysis is suppressed.

### SOLUTION TO PROBLEM

According to an embodiment of the present invention, there is provided a guanfacine hydrochloride preparation comprising guanfacine hydrochloride, one or more stability-improving additives selected from a group consisting of potassium dihydrogen phosphate, phosphoric acid, sodium dihydrogen phosphate anhydrous, carmellose, microcrystalline cellulose, carmellose calcium, low-substituted hydroxypropylcellulose, hypromellose phthalate, and glacial acetic acid; hypromellose acetate succinate; and one or more additives selected from a group consisting of aspartic acid, succinic acid, alginic acid, and glutamic acid.

0.5% by weight to 25% by weight of a stability-improving additive may be contained.

2% by weight to 35% by weight of hypromellose acetate succinate may be contained.

1% by weight to 15% by weight of one or more additives selected from the group consisting of aspartic acid, succinic acid, alginic acid, and glutamic acid may be contained.

According to an embodiment of the present invention, there is provided a use of glacial acetic acid in a guanfacine hydrochloride preparation. The guanfacine hydrochloride preparation contains guanfacine hydrochloride and glacial acetic acid.

The guanfacine hydrochloride preparation may contain 0.5% by weight to 25% by weight of glacial acetic acid.

### ADVANTAGEOUS EFFECTS OF INVENTION

According to an embodiment of the present invention, there is provided a guanfacine hydrochloride preparation in which an increase of related substances with time due to hydrolysis is suppressed.

### DESCRIPTION OF EMBODIMENTS

Hereinafter, a guanfacine hydrochloride preparation according to the present invention will be described. In addition, the guanfacine hydrochloride preparation of the present invention is not to be construed as being limited to the description contents of the following embodiments and examples.

As a result of studies by the present inventors, there is room for improvement in stability by an additive described in Patent Literature 1. Therefore, the present inventors have further studied and found that an increase of related substances of guanfacine hydrochloride can be suppressed by blending a specific additive. The present inventors have found that the use of hypromellose acetate succinate and one or more additives selected from a group consisting of aspartic acid, succinic acid, alginic acid, and glutamic acid in combination with a specific stability-improving additive can suppress the increase of related substances of guanfacine hydrochloride. In contrast to Patent Literature 1, which focuses on a specific organic acid and/or an enteric base, the present invention focuses on a specific stability-improving additive, and uses a combination of hypromellose acetate succinate and one or more additives selected from the group consisting of aspartic acid, succinic acid, alginic acid, and glutamic acid.

### [Guanfacine Hydrochloride Preparation]

The guanfacine hydrochloride preparation according to an embodiment of the present invention contains guanfacine hydrochloride, a stability-improving additive, hypromellose acetate succinate, and one or more additives selected from the group consisting of aspartic acid, succinic acid, alginic acid, and glutamic acid. In the present specification, the stability-improving additive is an additive capable of suppressing the increase of the related substances of guanfacine hydrochloride, and examples thereof include potassium dihydrogen phosphate, phosphoric acid, sodium dihydrogen phosphate anhydrous, carmellose, microcrystalline cellulose, carmellose calcium, low-substituted hydroxypropyl cellulose, hypromellose phthalate, and glacial acetic acid. These stability-improving additives can be contained in the guanfacine hydrochloride preparation alone or in a combination of two or more. In addition, in the present specification, L-aspartic acid may be selected as the aspartic acid.

The guanfacine hydrochloride preparation according to an embodiment of the present invention preferably contains one or more stability-improving additives selected from a group consisting of potassium dihydrogen phosphate, phosphoric acid, sodium dihydrogen phosphate anhydrous, carmellose, microcrystalline cellulose, carmellose calcium, low-substituted hydroxypropyl cellulose, hypromellose phthalate, glacial acetic acid, and hypromellose acetate succinate, and one or more additives selected from the group consisting of aspartic acid, succinic acid, alginic acid, and glutamic acid. Carmellose is particularly preferred as a stability-improving additive. In addition, aspartic acid, alginic acid, or glutamic acid is preferred as the additive to be added together with the stability-improving additive and the hypromellose acetate succinate.

The guanfacine hydrochloride preparation according to an embodiment of the present invention contains one or more pharmaceutically acceptable additives in addition to guanfacine hydrochloride, the stability-improving additive, hypromellose acetate succinate, and one or more additives selected from the group consisting of aspartic acid, succinic acid, alginic acid, and glutamic acid.

The guanfacine hydrochloride preparation according to an embodiment of the present invention contains 0.5% by weight to 25% by weight, preferably 0.9% by weight to 8% by weight of the stability-improving additive. The guanfacine hydrochloride preparation according to an embodiment of the present invention contains the above-described stability-improving additive within these ranges, thereby suppressing the increase of the related substances with time due to hydrolysis.

The guanfacine hydrochloride preparation according to an embodiment of the present invention contains 2% by weight to 35% by weight, preferably 6% by weight to 7% by weight of hypromellose acetate succinate.

The guanfacine hydrochloride preparation according to an embodiment of the present invention contains 1% by weight to 15% by weight, preferably 6% by weight to 10% by weight of one or more additives selected from the group consisting of aspartic acid, succinic acid, alginic acid, and glutamic acid.

The guanfacine hydrochloride preparation according to an embodiment of the present invention contains guanfacine hydrochloride and glacial acetic acid, whereby the increase of related substances with time due to hydrolysis can be suppressed. In this case, the guanfacine hydrochloride preparation can contain 0.5% by weight to 25% by weight of glacial acetic acid.

The guanfacine hydrochloride preparation according to an embodiment of the present invention can contain 1.14 mg, 2.28 mg, or 3.42 mg (1 mg, 2 mg, or 3 mg as guanfacine) of guanfacine hydrochloride per tablet. However, the content of guanfacine hydrochloride is not limited to these, and can be arbitrarily set within a range in which a therapeutic effect can be obtained.

Examples of one or more pharmaceutically acceptable additives contained in the guanfacine hydrochloride preparation according to an embodiment of the present invention can include but are not limited to excipients, disintegrants, sustained release agents, binders, lubricants, and colorants.

Examples of the excipient contained in the guanfacine hydrochloride preparation according to an embodiment of the present invention include but are not limited to one or more selected from mannitol, xylitol, dicalcium phosphate, calcium sulfate, starch, lactose, sucrose, dextrose, sorbitol, and cellulose powder. The guanfacine hydrochloride preparation according to an embodiment of the present invention contains 3% by weight to 90% by weight, preferably 10% by weight to 50% by weight of the excipient.

Examples of the disintegrant contained in the guanfacine hydrochloride preparation according to an embodiment of the present invention include but are not limited to one or more selected from starch, crospovidone, sodium starch glycolate, and croscarmellose sodium. The guanfacine hydrochloride preparation according to an embodiment of the present invention contains 0% by weight to 30% by weight, preferably 0.5% by weight to 20% by weight of the disintegrant.

The sustained-release agent contained in the guanfacine hydrochloride preparation according to an embodiment of the present invention is not particularly limited. The guanfacine hydrochloride preparation according to an embodiment of the present invention contains 15% by weight to 95% by weight, preferably 25% by weight to 70% by weight of the sustained release agent.

Examples of the binder contained in the guanfacine hydrochloride preparation according to an embodiment of the present invention can include but are not limited to one or more selected from polyvinylpyrrolidone, starch, methylcellulose, hydroxypropyl methylcellulose, sucrose solution, dextrose solution, acacia, tragacanth, and locust bean gum. The guanfacine hydrochloride preparation according to an embodiment of the present invention contains 0.2% by weight to 30% by weight, preferably 5% by weight to 15% by weight of the binder.

Examples of the lubricant contained in the guanfacine hydrochloride preparation according to an embodiment of the present invention can include but are not limited to one or more selected from talc, corn starch, silicon dioxide, sodium lauryl sulfate, magnesium stearate, calcium stearate, sodium stearate, stearic acid, sodium stearyl fumarate, hydrogenated cottonseed oil, and waxes (beeswax, carnauba wax, cetyl alcohol, glyceryl stearate, glyceryl palmitate, glyceryl behenate, hydrogenated vegetable oil, and stearyl alcohol). The guanfacine hydrochloride preparation according to an embodiment of the present invention contains 0.2% by weight to 20% by weight, preferably 5% by weight to 15% by weight of the lubricant.

Examples of the colorant contained in the guanfacine hydrochloride preparation according to an embodiment of the present invention can include but are not limited to one or more selected from blue No. 2 aluminum lake and yellow ferric oxide.

### [Evaluation of Stability]

In the present specification, the stability of the guanfacine hydrochloride containing pharmaceutical preparation can be assessed by measuring the amount of related substances using high-performance liquid chromatography (HPLC). 2,6-dichlorophenylacetic acid, which is hydrolysate, is known as a related substance of guanfacine hydrochloride.

### [Method for Producing Guanfacine Hydrochloride Containing Pharmaceutical Preparation]

The method for producing the guanfacine hydrochloride preparation according to the present invention is not particularly limited. In an embodiment, a direct tableting method can be used in which guanfacine hydrochloride, the stability-improving additive, hypromellose acetate succinate, one or more additives selected from the group consisting of aspartic acid, succinic acid, alginic acid, and glutamic acid, and one or more pharmaceutically acceptable additives are mixed and directly compressed.

### EXAMPLES

### [Comparative Example 1]

Guanfacine hydrochloride 17.1 g, silicified microcrystalline cellulose (PROSOLV HD90, JRS Pharma) 213.9 g, lactose hydrate (Dilactose (registered trademark) S, Freund Corporation) 113 g, crospovidone (Kollidon (registered trademark) CL-F, BASF) 6.00 g, methacrylic acid copolymer (Eudragit (registered trademark) L100-55, Evonik Roehm) 200 g, hydroxypropyl methylcellulose (Methocel (registered trademark) K4M, Colorcon, Inc.) 300 g, glyceryl behenate (Compritol (registered trademark) 888, GATTEFOSSE) 150 g, blue No. 2 aluminum lake 1.00 g, yellow ferric oxide 1.00 g were placed in a plastic bag and mixed. The obtained pharmaceutical composition was tableted using a rotary tableting machine (KIKUSUI SEISAKUSHO LTD) to obtain a guanfacine hydrochloride preparation of Comparative Example 1.

### [Reference Example 1]

Guanfacine hydrochloride 1.71 g, silicified microcrystalline cellulose (PROSOLV HD90, JRS Pharma) 21.39 g, lactose hydrate (Dilactose (registered trademark) S, Freund Corporation) 6.30 g, crospovidone (Kollidon (registered trademark) CL-F, BASF) 0.60 g, methacrylic acid copolymer (Eudragit (registered trademark) L100-55, Evonik Roehm) 20.0 g, hydroxypropyl methylcellulose (Methocel (registered trademark) K4M, Colorcon, Inc.) 30.0 g, glyceryl behenate (Compritol (registered trademark) 888, GATTEFOSSE) 15.0 g, blue No. 2 aluminum lake 0.10 g, yellow ferric oxide 0.10 g, potassium dihydrogen phosphate (NACALAI TESQUE, INC.) 5.00 g were placed in a plastic bag and mixed. The obtained pharmaceutical composition was tableted using the rotary tableting machine (KIKUSUI SEISAKUSHO LTD.) to obtain a guanfacine hydrochloride preparation of Reference Example 1.

### [Reference Example 2]

A guanfacine hydrochloride preparation of Reference Example 2 was obtained by the same production method as the production method of Reference Example 1 except that lactose hydrate (Dilactose (registered trademark) S, Freund Corporation) was changed to 8.80 g and potassium dihydrogen phosphate (NACALAI TESQUE, INC.) 2.50 g was added.

### [Reference Example 3]

A guanfacine hydrochloride preparation of Reference Example 3 was obtained by the same production method as the production method of Reference Example 1 except that lactose hydrate (Dilactose (registered trademark) S, Freund Corporation) was changed to 3.80 g and potassium dihydrogen phosphate (NACALAI TESQUE, INC.) 7.50 g was added.

### [Reference Example 4]

Guanfacine hydrochloride 1.37 g, silicified microcrystalline cellulose (PROSOLV HD90, JRS Pharma) 17.11 g, lactose hydrate (Dilactose (registered trademark) S, Freund Corporation) 7.04 g, crospovidone (Kollidon (registered trademark) CL-F, BASF) 0.48 g, methacrylic acid copolymer (Eudragit (registered trademark) L100-55, Evonik Roehm) 16.0 g, hydroxypropyl methylcellulose (Methocel (registered trademark) K4M, Colorcon, Inc.) 24.0 g, glyceryl behenate (Compritol (registered trademark) 888, GATTEFOSSE) 12.0 g, blue No. 2 aluminum lake 0.08 g, yellow ferric oxide 0.08 g, phosphoric acid (FUJIFILM Wako Pure Chemical Corporation) 2.00 g were placed in a plastic bag and mixed. The obtained pharmaceutical composition was tableted using the rotary tableting machine (KIKUSUI SEISAKUSHO LTD) to obtain a guanfacine hydrochloride preparation of Reference Example 4.

### [Reference Example 5]

A guanfacine hydrochloride preparation of Reference Example 5 was obtained by the same production method as the production method of Reference Example 4 except that lactose hydrate (Dilactose (registered trademark) S, Freund Corporation) was changed to 5.04 g and sodium dihydrogen phosphate anhydrous (FUJIFILM Wako Pure Chemical Corporation) 4.00 g was added instead of phosphoric acid.

### [Reference Example 6]

A guanfacine hydrochloride preparation of Reference Example 6 was obtained by the same production method as the production method of Reference Example 5 except that carmellose (NS-300 (registered trademark), NICHIRIN CHEMICAL INDUSTRIES, LTD.) 4.00 g was added instead of sodium dihydrogen phosphate anhydrous.

### [Reference Example 7]

A guanfacine hydrochloride preparation of Reference Example 7 was obtained by the same production method as the production method of Reference Example 5 except that lactose hydrate (Dilactose (registered trademark) S, Freund Corporation) was changed to 5.52 g, carmellose (NS-300 (registered trademark), NICHIRIN CHEMICAL INDUSTRIES, LTD.) 4.00 g was added instead of sodium dihydrogen phosphate anhydrous, and crospovidone was not added.

### [Reference Example 8]

A guanfacine hydrochloride preparation of Reference Example 8 was obtained by the same production method as the production method of Reference Example 4 except that lactose hydrate (Dilactose (registered trademark) S, Freund Corporation) was changed to 7.52 g, carmellose (NS-300 (registered trademark), NICHIRIN CHEMICAL INDUSTRIES, LTD.) 2.00 g was added instead of phosphoric acid, and crospovidone was not added.

### [Reference Example 9]

A guanfacine hydrochloride preparation of Reference Example 9 was obtained by the same production method as the production method of Reference Example 4 except that lactose hydrate (Dilactose (registered trademark) S, Freund Corporation) was changed to 8.72 g, carmellose (NS-300 (registered trademark), NICHIRIN CHEMICAL INDUSTRIES, LTD.) 0.80 g was added instead of phosphoric acid, and crospovidone was not added.

### [Reference Example 10]

A guanfacine hydrochloride preparation of Reference Example 10 was obtained by the same production method as the production method of Reference Example 5 except that microcrystalline cellulose (CEOLUS UF-702, Asahi Kasei Corp.) 4.00 g was added instead of sodium dihydrogen phosphate anhydrous.

### [Reference Example 11]

A guanfacine hydrochloride preparation of Reference Example 11 was obtained by the same production method as the production method of Reference Example 5 except that carmellose calcium (E.C.G-505 (registered trademark), NICHIRIN CHEMICAL INDUSTRIES, LTD.) 4.00 g was added instead of sodium dihydrogen phosphate anhydrous.

### [Reference Example 12]

A guanfacine hydrochloride preparation of Reference Example 12 was obtained by the same production method as the production method of Reference Example 5 except that low-substituted hydroxypropylcellulose (L-HPC (registered trademark) LH-21, Shin-Etsu Chemical Co., Ltd.) 4.00 g was added instead of sodium dihydrogen phosphate anhydrous.

### [Reference Example 13]

A guanfacine hydrochloride preparation of Reference Example 13 was obtained by the same production method as that of Reference Example 5 except that hypromellose phthalate (HPMCP (registered trademark) 55, Shin-Etsu Chemical Co., Ltd.) 4.00 g was added instead of sodium dihydrogen phosphate anhydrous.

### [Example 1]

A guanfacine hydrochloride preparation of Example 1 was obtained by the same production method as the production method of Reference Example 5 except that glacial acetic acid (Daicel Corporation) 4.00 g was added instead of sodium dihydrogen phosphate anhydrous.

### [Example 2]

Guanfacine hydrochloride 1.14 g, microcrystalline cellulose (CEOLUS (registered trademark) UF-702, Asahi Kasei Corp.) 22.0 g, lactose hydrate (Dilactose (registered trademark) S, Freund Corporation) 28.86 g, methacrylic acid copolymer (Eudragit (registered trademark) L100-55, Evonik Roehm) 35.0 g, hydroxypropyl methyl cellulose (Methocel (registered trademark) K4M, Colorcon, Inc.) 20.0 g, glyceryl behenate (Compritol (registered trademark) 888, GATTEFOSSE) 20.0 g, carmellose (NS-300 (registered trademark), NICHIRIN CHEMICAL INDUSTRIES, LTD.) 3.0 g, L-aspartic acid (FUJIFILM Wako Pure Chemical Corporation) 10.0 g, hypromellose acetate succinate (AQOAT (registered trademark) AS-LF, Shin-Etsu Chemical Co., Ltd.) 10.0 g were placed in a plastic bag and mixed. The obtained pharmaceutical composition was tableted using the rotary tableting machine (KIKUSUI SEISAKUSHO LTD) to obtain a guanfacine hydrochloride preparation of Example 2.

### [Example 3]

A guanfacine hydrochloride preparation of Example 3 was obtained by the same production method as the production method of Example 2 except that succinic acid (Tokyo Chemical Industry Co., Ltd.) 10.0 g was added instead of L-aspartic acid.

### [Example 4]

A guanfacine hydrochloride preparation of Example 4 was obtained by the same preparation method as the production method of Example 2 except that lactose hydrate was changed to 23.86 g and alginic acid (NACALAI TESQUE, INC.) 15.0 g was added instead of L-aspartic acid.

### [Example 5]

A guanfacine hydrochloride preparation of Example 5 was obtained by the same production method as the production method of Example 2 except that glutamic acid (FUJIFILM Wako Pure Chemical Corporation) 10.0 g was added instead of L-aspartic acid.

### [Comparative Example 2]

A guanfacine hydrochloride preparation of Comparative Example 2 was obtained by the same production method as the production method of Reference Example 5 except that citric acid monohydrate (NACALAI TESQUE, INC.) 4.00 g was added instead of sodium dihydrogen phosphate anhydrous.

### [Comparative Example 3]

A guanfacine hydrochloride preparation of Comparative Example 3 was obtained by the same production method as the production method of Reference Example 5 except that dipotassium hydrogen phosphate (NACALAI TESQUE, INC.) 4.00 g was added instead of sodium dihydrogen phosphate anhydrous.

### [Comparative Example 4]

A guanfacine hydrochloride preparation of Comparative Example 4 was obtained by the same production method as the production method of Reference Example 5 except that croscarmellose sodium (Ac-Di-Sol (registered trademark), DuPont Nutrition Ireland) 4.00 g was added instead of sodium dihydrogen phosphate anhydrous.

### [Comparative Example 5]

A guanfacine hydrochloride preparation of Comparative Example 5 was obtained by the same production method as the production method of Reference Example 5 except that succinic acid (Tokyo Chemical Industry Co., Ltd.) 4.00 g was added instead of sodium dihydrogen phosphate anhydrous.

### [pH Measurement]

One tablet of the guanfacine hydrochloride preparations of Reference Examples 1 to 13, Examples 1 to 5, and Comparative Examples 1 to 5 was pulverized, and 10 ml of water was added. The mixture was mixed with Voltex for 10 seconds, and sonicated for 15 minutes to extract the components contained in the guanfacine hydrochloride preparation. The extract was roughly filtered with a Kimwipe to remove gel-like residues, and then the pH of the filtrate was measured. Measurement results are shown in Table 1.

### [Evaluation of Stability]

The guanfacine hydrochloride preparations of Reference Examples 1 to 13, Examples 1 to 5, and Comparative Examples 1 to 5 were stored at 60°C and 60% relative humidity open conditions for 1 week or 2 weeks. The initial value before storing (initial) and the amount (%) of the related substance (hydrolysate) at the time of storing were measured. One tablet before storage (immediately after production) and after storage was pulverized using an agate mortar, and about 8 ml of acetonitrile : water admixture (4 : 1) was added and stirred. In addition, acetonitrile / water (4 : 1) was added to make exactly 10 ml. This solution was filtered through a membrane filter with a pore size of 0.45 µm or less, and 3 ml or more of the first filtrate was removed, and the next filtrate was used as a sample solution.

The amount of related substances was measured by an HPLC method, taking exactly 2 µl of the above sample solution. By the area percentage method, the sum of the peak areas of guanfacine and related substances derived from guanfacine obtained on a chromatogram was taken as 100, and the amount (%) of 2,6-dichlorophenylacetic acid, which is a main related substance derived from guanfacine hydrochloride, was calculated from the ratio of the peak areas.

The values of hydrolysate of guanfacine hydrochloride are shown in Table 1.

**[Table 1]**

| | Comparative Example 1 | Reference Example 1 | Reference Example 2 | Reference Example 3 | Reference Example 4 |
|---|---|---|---|---|---|
| pH | 5.78 | 4.25 | 4.55 | 4.66 | 2.98 |
| Initial | 0.06 | 0.06 | N.D. | 0.02 | N.D. |
| 60°C, 60%RH, open 1W | 0.42 | 0.15 | 0.13 | 0.15 | 0.09 |
| 60°C, 60%RH, open 2W | 0.75 | 0.31 | 0.27 | 0.28 | 0.22 |

| | Reference Example 5 | Reference Example 6 | Reference Example 7 | Reference Example 8 | Reference Example 9 |
|---|---|---|---|---|---|
| pH | 4.53 | 4.18 | 4.53 | 4.44 | 4.57 |
| Initial | N.D. | N.D. | N.D. | N.D. | N.D. |
| 60°C, 60%RH, open 1W | 0.13 | 0.04 | 0.03 | 0.04 | 0.08 |
| 60°C, 60%RH, open 2W | 0.31 | 0.09 | 0.07 | 0.07 | 0.11 |

| | Reference Example 10 | Reference Example 11 | Reference Example 12 | Reference Example 13 | Example 1 |
|---|---|---|---|---|---|
| pH | 4.72 | 5.61 | 5.68 | 4.37 | 3.39 |
| Initial | N.D. | N.D. | N.D. | N.D. | N.D. |
| 60°C, 60%RH, open 1W | 0.12 | 0.17 | 0.17 | 0.13 | 0.16 |
| 60°C, 60%RH, open 2W | 0.27 | 0.33 | 0.26 | 0.20 | 0.32 |

| | Example 2 | Example 3 | Example 4 | Example 5 | Comparative Example 2 |
|---|---|---|---|---|---|
| pH | 3.51 | 3.30 | 3.52 | 3.55 | 2.98 |
| Initial | N.D. | N.D. | N.D. | N.D. | N.D. |
| 60°C, 60%RH, open 1W | 0.17 | 0.34 | 0.15 | 0.14 | 0.28 |
| 60°C, 60%RH, open 2W | 0.22 | 0.50 | 0.12 | 0.20 | 0.67 |

| | Comparative Example 3 | Comparative Example 4 | Comparative Example 5 | | |
|---|---|---|---|---|---|
| pH | 6.90 | 6.12 | 3.33 | | |
| Initial | N.D. | N.D. | N.D. | | |
| 60°C, 60%RH, open 1W | 2.13 | 0.54 | 0.28 | | |
| 60°C, 60%RH, open 2W | 5.28 | 1.05 | 0.75 | | |

From the results shown in Table 1, in the guanfacine hydrochloride preparations of Comparative Example 3 in which pH was 6.90, and Comparative Example 4 in which pH was 6.12, a significant increase in the hydrolysate of guanfacine hydrochloride was observed. On the other hand, due to the effect of the stability-improving additive, the amount of hydrolysate of guanfacine hydrochloride was reduced in the guanfacine hydrochloride preparations of Reference Examples 1 to 5, 10 to 13, and Example 1 with a lower pH than in Comparative Example 1 in which the additive is not contained. However, in the guanfacine hydrochloride preparations of Comparative Example 2 in which pH was 2.98, and of Comparative Example 5 in which pH was 3.33, an increase in the hydrolysate of guanfacine hydrochloride was observed. In addition, in the guanfacine hydrochloride preparations of Reference Examples 6 to 9 containing carmellose as the stability-improving additive, an increase in the hydrolysate of guanfacine hydrochloride was significantly suppressed.

Furthermore, the amount of hydrolysate of guanfacine hydrochloride was reduced in the guanfacine hydrochloride preparations of Example 2, which contains hypromellose acetate succinate in addition to carmellose, and further contains L-aspartic acid, Example 4, which further contains alginic acid, and Example 5, which further contains glutamic acid. In addition, in the guanfacine hydrochloride preparation of Comparative Example 5 containing succinic acid, although an increase in the hydrolysate of guanfacine hydrochloride was observed, in the guanfacine hydrochloride preparation of Example 3 containing hypromellose acetate succinate along with carmellose in addition to succinic acid, the amount of hydrolysate of guanfacine hydrochloride was significantly reduced compared with the guanfacine hydrochloride preparation of Comparative Example 5.

From the above, it was clarified that, in the guanfacine hydrochloride preparation, it is possible to suppress the increase of related substances by containing hypromellose acetate succinate and one or more additives selected from the group consisting of aspartic acid, succinic acid, alginic acid, and glutamic acid in addition to the stability-improving additive.

## Claims

1. A guanfacine hydrochloride preparation comprising:
guanfacine hydrochloride;
one or more stability-improving additives selected from a group consisting of potassium dihydrogen phosphate, phosphoric acid, sodium dihydrogen phosphate anhydrous, carmellose, microcrystalline cellulose, carmellose calcium, low-substituted hydroxypropylcellulose, hypromellose phthalate, and glacial acetic acid;
hypromellose acetate succinate; and
one or more additives selected from a group consisting of aspartic acid, succinic acid, alginic acid, and glutamic acid.

2. The guanfacine hydrochloride preparation according to claim 1, wherein
0.5% by weight to 25% by weight of the stability-improving additive is contained.

3. The guanfacine hydrochloride preparation according to claim 1, wherein
2% by weight to 35% by weight of the hypromellose acetate succinate is contained.

4. The guanfacine hydrochloride preparation according to claim 1, wherein
1% by weight to 15% by weight of the one or more additives selected from the group consisting of the aspartic acid, succinic acid, alginic acid, and glutamic acid is contained.

5. A use of glacial acetic acid in the guanfacine hydrochloride preparation, wherein
the guanfacine hydrochloride preparation contains guanfacine hydrochloride and glacial acetic.

6. The use of glacial acetic acid according to claim 5, wherein
the guanfacine hydrochloride preparation contains 0.5% by weight to 25% by weight of the glacial acetic acid.
